## Europäisches Patentamt

## European Patent Office

## Office européen des brevets

(19)

(11) Numéro de publication: **0 183 752**

**B1**

(12) **FASCICULE DE BREVET EUROPÉEN**

(45) Date de publication du fascicule du brevet:
**15.11.89**

(51) Int. Cl.⁴: **A 41 B 9/00, A 41 B 13/02**

(21) Numéro de dépôt: **85902553.8**

(22) Date de dépôt: **13.05.85**

(86) Numéro de dépôt international:
**PCT/FR 85/00114**

(87) Numéro de publication internationale:
**WO 85/05254 (05.12.85 Gazette 85/26)**

(54) **CULOTTE A CEINTURE ELASTIQUE, ET SON PROCEDE DE FABRICATION.**

(30) Priorité: **16.05.84 FR 8407909**
**28.06.84 BE 213234**
**30.10.84 BE 213923**

(43) Date de publication de la demande:
**11.06.86 Bulletin 86/24**

(45) Mention de la délivrance du brevet:
**15.11.89 Bulletin 89/46**

(84) Etats contractants désignés:
**AT BE CH DE FR GB IT LI LU NL SE**

(56) Documents cité:
**EP-A-0 048 011**
**FR-A-2 140 965**
**FR-A-2 177 425**
**FR-A-2 517 524**
**GB-A-2 072 491**
**US-A-4 407 284**

(73) Titulaire: **PEAUDOUCE, 59, Rue de la Vignette,
F-59126 Linselles (FR)**

(72) Inventeur: **HOOREMAN, Bernard, 4, allée des
Jonquilles, F-59917 Wervicq- Sud (FR)**
Inventeur: **KAHLE, Didier, 5, allée des Coqueleux,
F-59910 Bondue (FR)**

(74) Mandataire: **Casalonga, Axel, BUREAU D.A.
CASALONGA - JOSSE Morassistrasse 8, D-8000
Munich 5 (DE)**

## Description

La présente invention se rapporte à une culotte en forme de slip à ceinture élastique réalisée à partir d'une pièce plan de matériau souple, de forme générale rectangulaire et d'éléments élastiques fixés à cette pièce, ainsi qu'à un procédé de fabrication d'une telle culotte.

La fabrication traditionnelle d'articles d'habillement du type culotte, qu'il s'agisse de culotte en matériaux textiles ou d'articles d'hygiène tels que par exemple des couches-culottes pour enfants ou adultes incontinents, nécessite de nombreuses opérations qui sont guère adaptées à une fabrication en continu à grande cadence. Des problèmes se posent en particulier pour la réalisation de culottes à ceintures élastiques. En effet, pour permettre une fabrication en continu des culottes à partir d'un matériau en forme de bande, sans interruption du mouvement d'avancement de la bande, des éléments élastiques de ceinture devraient être rapportés ou formés dans le sens d'avancement de la bande, alors que suivant les procédés usuels de fabrication, les culottes sont formées dans la bande de matériau de manière que les parties de ceinture soient orientées perpendiculairement à la direction d'avancement de la bande.

La fabrication de couches-culottes comportant des éléments élastiques est décrite, par exemple, dans les documents EP-A-0 091 316, EP-A-0 145 080, EP-A-0 157 649, US-A-4 227 952, US-A-4 297 157, mais il s'agit là de couches-culottes de structure classique, c'est-à-dire ouvertes, et non pas fermées, en forme de slip, et les éléments élastiques sont prévus dans la zone d'entre-jambes. Par ailleurs, il est connu par le document US-A-4 407 284, de prévoir sur une telle couche-culotte ouverte des éléments élastiques à la fois dans la zone d'entre-jambes et dans la zone de ceinture. Par le document GB-A-2 072 491, on connaît une couche-culotte du type ouvert dont les moyens de fermeture ou d'attache sont constitués par deux bandes élastiques susceptibles d'être fixées de façon amovible à la couche-culotte. Enfin, on connaît par les documents FR-A-2 140 965, FR-A-2 177 425, FR-A2-2 517 524 et EP-A-0 048 011 des procédés de fabrication en continu de culottes ou couches-culottes en forme de slip à ceinture élastique à partir d'une bande munie d'éléments élastiques longitudinaux, mais ces procédés impliquent un certain nombre d'opérations (repliage de la bande sur elle-même, tronçonnage transversal de la bande, assemblage des bords transversaux des tronçons de bande repliés) qui compliquent la fabrication et font que ces procédés sont difficilement applicables à des articles d'hygiène devant être fabriqués à grande cadence tels que des couches-culottes.

La présente invention a pour objet une culotte à ceinture élastique, de conception simple, se prêtant à une fabrication en continu, à grande cadence.

L'invention a également pour objet un procédé de fabrication en continu de culottes à ceinture élastique à partir d'un matériau en forme de bande, procédé suivant lequel toutes les opérations et notamment celles concernant la pose d'éléments élastiques de ceinture peuvent être réalisées en continu, sans interruption du mouvement d'avancement de la bande de matériau.

La culotte conforme à l'invention en forme de slip à ceinture élastique, est réalisée à partir d'une pièce plane de matériau souple, de forme générale rectangulaire. La culotte comprend deux éléments élastiques s'étendant, lorsque ladite pièce de matériau souple est disposée à plat, à l'état tendu le long des deux bords longitudinaux opposés de ladite pièce. Les deux extrémités de chacun desdits éléments élastiques sont solidaires de ladite pièce uniquement en des endroits situés au voisinage des deux extrémités transversales de cette dernière. Ainsi, les deux éléments élastiques délimitent avec ladite pièce, lorsque la culotte en forme de slip se trouve en position d'utilisation, deux ouvertures de passage pour les jambes et forment deux éléments élastiques de ceinture à hauteur de la taille.

Sur la culotte conforme à l'invention, les deux éléments élastiques de ceinture peuvent être constitués par des bandes rapportées sur la pièce de matériau souple, uniquement leurs deux extrémités étant fixées sur ladite pièce.

Suivant un autre mode de réalisation de la culotte conforme à l'invention, les deux éléments élastiques de ceinture sont constitués par deux bandes longitudinales délimitées dans le matériau de ladite pièce par deux découpes longitudinales partielles ménagées dans ladite pièce, entre les deux extrémités transversales de cette dernière, en retrait vers l'intérieur par rapport aux bords longitudinaux opposés de ladite pièce.

Les éléments élastiques sont avantageusement fixés à la pièce de matériau souple en onglet par rapport aux coins de cette pièce.

Le procédé conforme à l'invention de fabrication en continu de culottes en forme de slip à ceinture élastique consiste, suivant un mode de réalisation, à:

- alimenter en continu, à l'état tendu, au moins une bande d'un matériau souple,
- alimenter en continu, parallèlement au sens de défilement de la bande, de part et d'autre de l'axe médian longitudinal de cette dernière, des éléments élastiques à l'état tendu,
- déterminer une longueur élémentaire de bande correspondant à la longueur nécessaire à la confection d'une culotte, la bande étant alors considérée comme formant une succession d'ébauches,
- solidariser de la bande à l'état tendu, les éléments élastiques à l'état tendu dans le sens de défilement de la bande, cette solidarisation s'effectuant uniquement en des endroits proches de l'extrémité arrière et de l'extrémité avant de chaque ébauche,
- découper la bande et les éléments élastiques dans le sens transversal, dans la zone de séparation des ébauches successives.

Le procédé conforme à l'invention de fabrication en continu de culottes en forme de slip à ceinture élastique, suivant un autre mode de réalisation, consiste à:

- alimenter en continu, à l'état tendu, au moins une bande de matériau souple,
- alimenter en continu parallèlement au sens de défilement de la bande, de part et d'autre de l'axe médian longitudinal de cette dernière, légèrement en retrait par rapport aux bords longitudinaux de la bande, des éléments élastiques à l'état tendu,

- déterminer une longueur élémentaire de bande correspondant à la longueur nécessaire à la confection d'une culotte, la bande étant alors considérée comme formant une succession d'ébauches,
- solidariser en continu de la bande à l'état tendu, les éléments élastiques à l'état tendu dans le sens de défilement de la bande,
- former dans la bande, à hauteur de chaque ébauche, entre les deux extrémités de cette dernière, en retrait vers l'intérieur par rapport auxdits élastiques, deux découpes partielles symétriques orientées généralement suivant la direction longitudinale de la bande et ayant une longueur légèrement inférieure à la longueur de l'ébauche, et
- découper la bande et les élastiques dans le sens transversal, dans la zone de séparation des ébauches successives.

De préférence, les éléments élastiques sont solidarisés avec la bande, à chaque coin d'une ébauche, sensiblement en onglet, c'est-à-dire sensiblement perpendiculairement à la bissectrice du coin considéré.

Suivant un autre mode de réalisation du procédé conforme à l'invention, on forme dans la bande, dans les zones d'extrémité avant et arrière de chaque ébauche, de part et d'autre de l'axe médian longitudinal de la bande des découpes en coin pour former des pointes aux emplacements des coins de la partie d'ébauche destinée à constituer la culotte,
on replie sur la face supérieure de l'ébauche les pointes ainsi formées,
on solidarise les éléments élastiques à l'état tendu de la face apparente des pointes repliées,
et on découpe dans la bande, dans chaque ébauche, la forme définitive de la partie de matériau souple constituant la culotte.

Pour la fabrication d'une couche-culotte, il peut être avantageux de solidariser de la bande, sur une partie de la longueur de chaque ébauche, sensiblement à mi-distance entre les extrémités de chaque ébauche, des longueurs limitées d'élastiques à l'état tendu, disposés généralement suivant la direction longitudinale de la bande, au voisinage des bords longitudinaux de la partie centrale de matériau souple constituant la culotte.

Suivant un autre mode de réalisation, on rabat les bords longitudinaux de la bande, pourvus d'élastiques et situés à l'extérieur des découpes, sur la partie centrale de l'ébauche, et on solidarise en onglet, de la partie centrale de l'ébauche, les parties des rabats situées entre les extrémités des découpes et les bords transversaux correspondants de l'ébauche.

En se référant aux dessin annexés, on va décrire ci-après plus en détail, à titre d'exemples illustratifs et non limitatifs, plusieurs modes de réalisation de l'invention; sur le dessin:

- la figure 1 est une vue en perspective d'un mode de réalisation simple d'une culotte suivant l'invention, à l'état tendu;
- la figure 2 est une vue également en perspective de la culotte de la figure 1, après détente des éléments élastiques;
- les figures 3 et 4 sont des vues en perspective de deux autres modes de réalisaton de la culotte conforme à l'invention, à l'état tendu;
- les figures 5a à 5e montrent, en perspective et à l'état tendu, un mode de réalisation préféré d'une culotte suivant l'invention, à ses différents stades de fabrication;
- la figure 5f représente la culotte obtenue à la suite de l'étape 5e, après rétraction des éléments élastiques;
- les figures 5g et 5h sont des vues partielles en perspective d'une variante de réalisation des étapes illustrées sur les figures 5d et 5e;
- la figure 6 illustre de manière schématique un procédé de fabrication en continu de couches-culottes suivant l'invention, pourvues de coussins absorbant;
- la figure 7 illustre, de manière schématique, une variante du procédé de la figure 6;
- les figures 8a et 8b représentent des phases de pliage successives d'une couche-culotte selon les figures 5e, 5h, ou d'une couche-culotte obtenue par le procédé suivant la figure 7;
- la figure 9 montre schématiquement les étapes d'un procédé de fabrication conforme à l'invention, permettant l'obtention d'un autre type de culottes;
- la figure 10 est une vue explosée d'une couche-culotte conforme à l'invention de type simple, les différents éléments constitutifs étant représentés à l'état tendu.

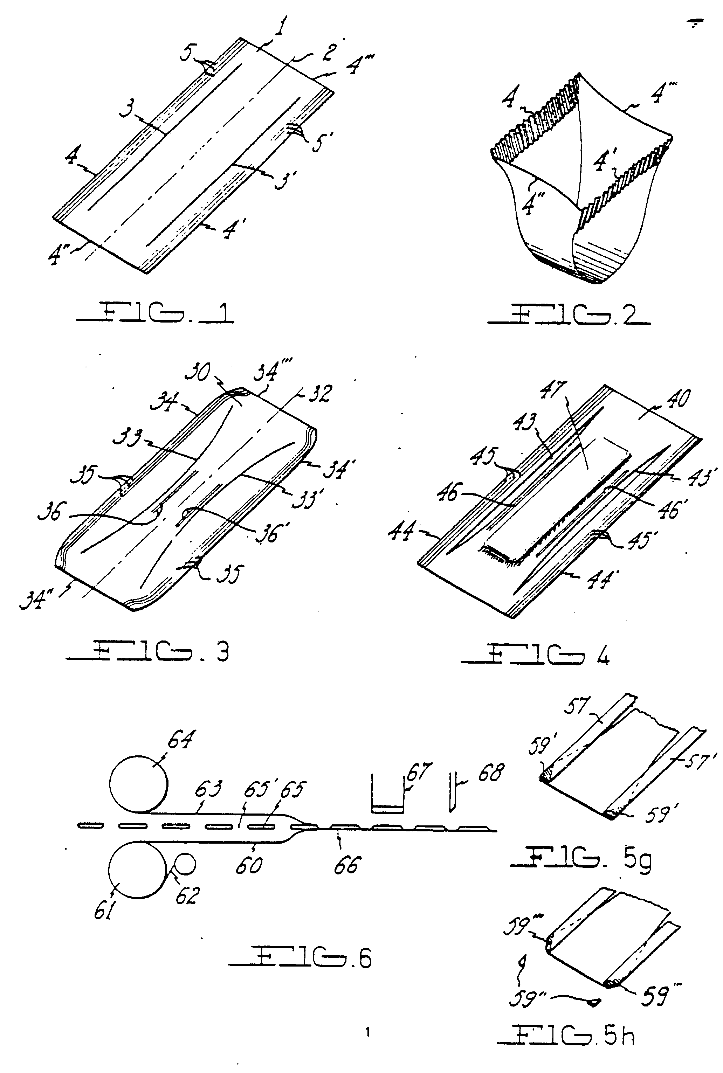

En se reportant plus particulièrement à la figure 1, on reconnaît qu'une culotte suivant l'invention, de conception extrêmement simple, comprend essentiellement une feuille 1, plane, souple, rectangulaire et donc symétrique par rapport à son axe longitudinal 2, dans laquelle sont formées des découpes longitudinales partielles 3, 3', sous forme de fentes rectilignes, au moins sensiblement symétriques par rapport à l'axe 2. Sur la feuille sont fixés, dans des zones crises entre les bords longitudinaux 4, 4' et les découpes 3, 3', des élastiques 5, 5' s'étendant longitudinalement. Les élastiques sont solidarisés de la feuille à l'état tendu, sur la longueur des bords longitudinaux 4, 4', de toutes manières convenables, par exemple par collage, soudure ou couture.

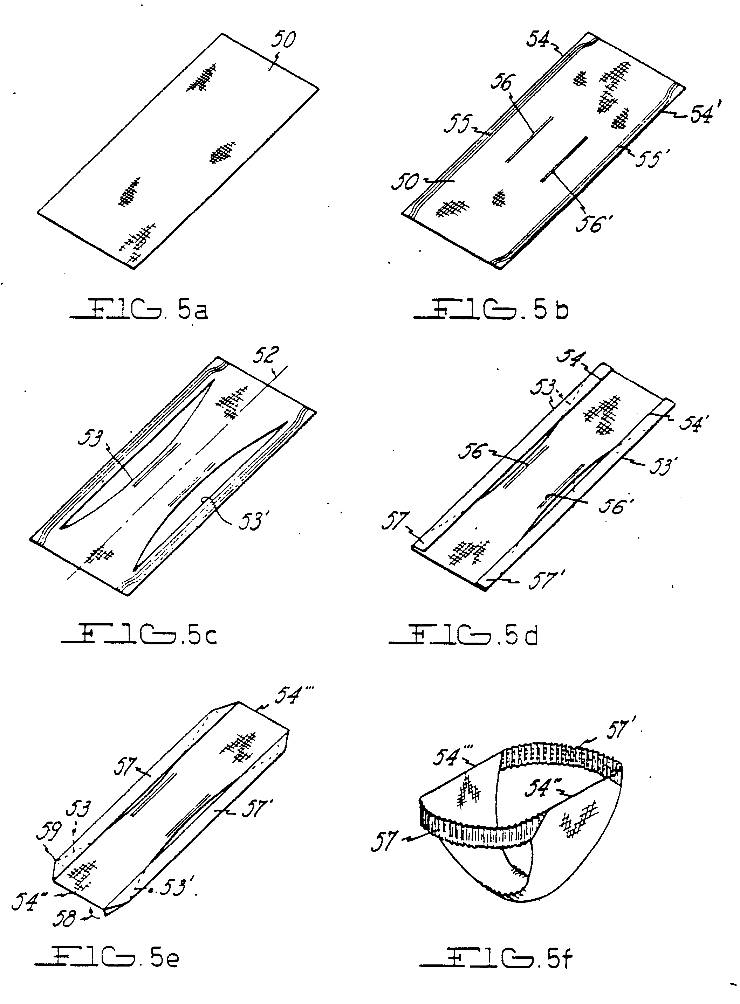

Lorsque la pièce plane ainsi constituée, avec les élastiques 5, 5', est libérée de toute contrainte, les élastiques 5, 5' se contractant, provoquant un plissage des bords longitudinaux 4, 4' de l'article, et amenant l'article dans la forme représentée en figure 2.

Les proportions de la pièce plane (longueur par rapport à la largeur), et l'allongement des élastiques lors de leur solidarisation avec la feuille, sont déterminées pour que, lors de la réduction de périmètre apparent résultant du plissage des bords longitudinaux, la périphérie de l'article obtenu (bords latéraux 4, 4' élastiquement rétractés et bords transversaux 4'', 4''' non élastiques) constitue une ceinture élastique correspondant à la taille de la culotte considérée.

Une telle culotte peut être réalisée en continu suivant un procédé consistant à alimenter en continu, à l'état tendu, au moins une bande d'un matériau souple, à alimenter en continu parallèlement au sens de défilement de la bande, de part et d'autre de l'axe médian longitudinal de la bande, des élastiques à l'état tendu, à déterminer une longueur élémentaire de bande correspondant à la longueur nécessaire à la confection d'un article, la bande étant alors considérée comme une succession d'ébauches d'articles, à solidariser de la bande à l'état tendu des élastiques à l'état tendu disposés dans le sens de défilement de la bande, à former dans la bande, à hauteur de chaque ébauche, entre les deux extrémités transversales de cette dernière, en retrait vers l'intérieur par rapport auxdits élastiques, deux découpes symétriques par rapport à l'axe médian longitudinal de la bande, lesdites découpes étant orientées d'une manière générale suivant la direction de cet axe, et à découper transversalement la bande et les élastiques fixés à cette dernière, dans le sens transversal, dans la zone de séparation des ébauches successives.

Suivant la variante de la figure 3, on forme dans une pièce plane oblongue 30 de matériau souple des découpes partielles 33, 33' sous forme de fentes curvilignes, généralement symétriques par rapport à l'axe longitudinal 32. Des élastiques 35, 35' à l'état tendu sont solidarisés de la pièce 30 à l'état tendu, dans les zones adjacentes aux bords longitudinaux 34, 34', pour constituer une ceinture élastique. Ces élastiques sont posés suivant une trajectoire généralement rectiligne, s'incurvant aux extrémités de la pièce plane. Ces dispositions permettent de réduire la largeur des bords transversaux 34'', 34''' dépourvue d'élastiques. D'autres élastiques 36, 36' sont solidarisés de la pièce plane 30, également à l'état tendu, dans les zones adjacentes aux bords internes des découpes 33, 33', pour assurer un serrage sur les jambes.

En se reportant à la variante de réalisation de la figure 4, on reconnaît une pièce plane 40 constituée d'un matériau multicouche, pourvue de découpes 43, 43' avec enlèvement de matière. Ces découpes comprennent chacune une découpe rectiligne et une découpe curviligne. Comme dans le mode de réalisation illustré à la figure 3, des élastiques longitudinaux 45, 45' à l'état tendu sont rendus solidaires de la pièce 40, dans la zone des bords longitudinaux 44, 44', sur toute la longueur, et des élastiques 46, 46' à l'état tendu sont rendus solidaires de la pièce 40 dans les zones adjacentes aux bords internes des découpes 43, 43'. En outre, le matériau multicouche de la pièce plane 40 comporte un coussin absorbant 47 dans la zone située entre les découpes 43, 43'.

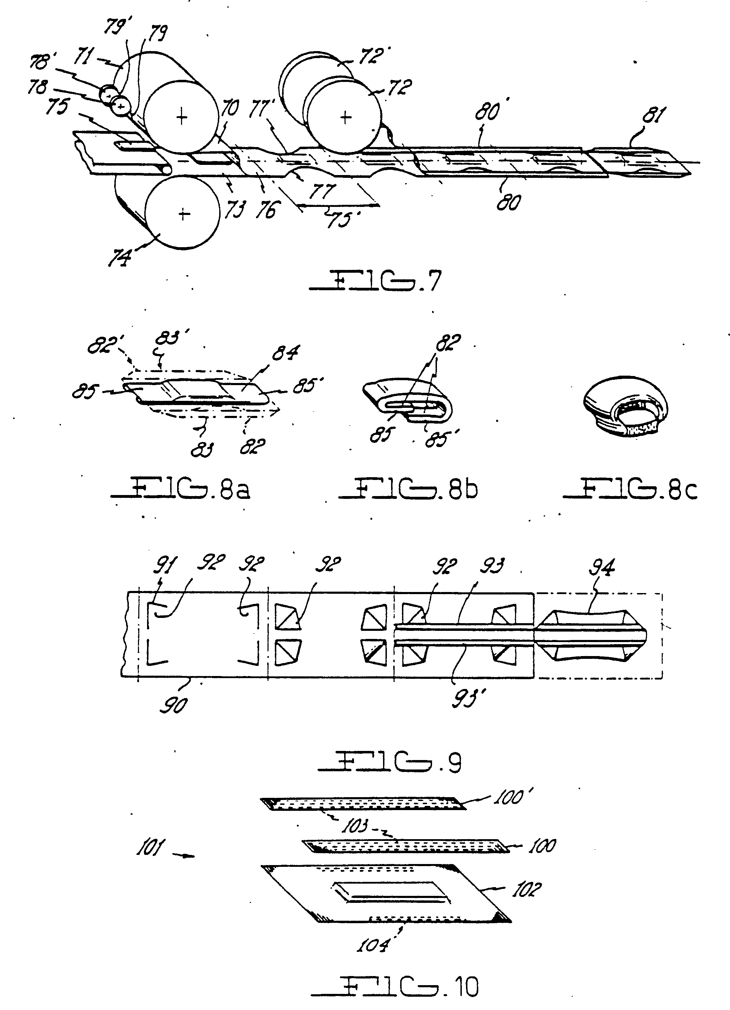

La figure 6 illustre schématiquement la mise en oeuvre d'un procédé suivant l'invention pour la réalisation d'une couche-culotte illustrée à la figure 4.

Suivant ce mode de mise en oeuvre, une bande de matériau souple 60, de préférence une matière plastique imperméable à l'humidité, destinée à constituer la feuille extérieure de la couche-culotte, est alimentée en continu à partir d'une bobine 61. Pour l'ensemble des opérations suivantes, on détermine une longueur élémentaire de bande correspondant à la longueur d'une ébauche nécessaire à la réalisation d'une couche-culotte. Des élastiques 62, à l'état tendu, sont fixés à la bande, en continu dans la zone des bords longitudinaux de celle-ci, et de manière discontinue dans chaque ébauche, à hauteur des zones prévues pour les découpes et dans les régions du bord interne de celles-ci. La bande 60 est maintenue à l'état tendu.

Simultanément, une seconde bande 63 d'un matériau perméable à l'humidité, par exemple un non-tissé, est alimentée en continu à partir d'une bobine 64, et des coussins 65 de matière absorbante sont amenés de manière discontinue, entre les deux bandes 60 et 63, dans la zone de l'axe longitudinal médian de ces dernières, lesdits coussins étant séparés par des intervalles 65' nécessaires pour qu'un coussin 65, dont la longueur est inférieure à la longueur d'une ébauche, se trouve toujours au même endroit d'une ébauche. Les deux bandes 60, 63 et lesdits coussins 65 sont ensuite solidarisés sous la forme d'un matériau composite 66 maintenu à l'état tendu. Un premier organe de découpage 67, agissant de manière périodique, est prévu pour réaliser les découpes longitudinales partielles dans le matériau composite tendu, à hauteur de chaque ébauche. Ensuite, un second organe de découpage 68, disposé transversalement au matériau composite en forme de bande, réalise, également périodiquement, la séparation des différentes couches-culottes dans cette bande. Après cette séparation, la tension qui maintenait le matériau composite à l'état tendu cesse, de sorte que les élastiques fixés à l'état tendu au matériau composite se contractent et assurent ainsi la mise en forme des couches-culottes.

Les figures 5a à 5f illustrent un mode de réalisation d'une autre culotte suivant l'invention, au cours de ses diverses étapes de fabrication.

La culotte est formée à partir d'une feuille rectangulaire 50 de matériau souple (figure 5a). Au cours de l'étape suivante (figure 5b), cette feuille 50, maintenue à l'état tendu, est munie, dans les zones proches de ses bords longitudinaux 54, 54', d'élastiques 55, 55' à l'état tendu, et aussi, dans la zone prévue pour les découpes, dans la région du bord interne de celle-ci, d'élastiques 56, 56' également à l'état tendu. Les élastiques 55, 55' et 56, 56' sont fixés par exemple par collage.

Ensuite (figure 5c) tout en maintenant à l'état tendu la feuille 50 avec les élastiques 55, 55', 56, 56' qui s'y trouvent fixés, on forme dans la feuille 50 des découpes longitudinales partielles 53, 53' au moins sensiblement symétriques par rapport à l'axe longitudinal médian 52.

On forme ensuite (figure 5d), la feuille 50 restant à l'état tendu, des rabats 57, 57' par pliage des zones des bords situées à l'extérieur des découpes 53, 53' sur la partie centrale de la feuille.

Enfin (figure 5e), la feuille 50 étant maintenue à l'état tendu, on replie sur la partie centrale, suivant la flèche 58, la partie du rabat située entre l'extrémité 59 de la découpe 53, 53' et le bord transversal correspondant 54'' de la feuille, et on solidarise cette partie repliée, par exemple par collage, soudage, etc., de la partie centrale.

Lorsqu'on libère la culotte ainsi constituée, cette dernière prend la forme illustrée sur la figure 5f, sur l'effet de la contraction élastique des zones 57, 57' comportant les élastiques 55, 55'.

Le pliage illustré sur les figures 5d et 5e présente l'avantage, d'une part, par un choix judicieux des dimensions des rabats, d'amener les zones élastiques 57, 57' des bords longitudinaux 54, 54' dans le prolongement des bords transversaux 54'', 54''', et d'autre part, d'assurer un renforcement de l'extrémité 59 des découpes 53, 53', ainsi qu'une certaine réduction du périmètre apparent de l'article obtenu. On obtient ainsi une culotte d'un aspect plus traditionnel.

Suivant la variante de réalisation représentée sur les figures 5g et 5h, on réalise par collage ou par soudage une jonction triangulaire 59' entre l'extrémité des rabats 57, 57' et la partie centrale, puis on découpe la partie extrême 59'' de cette surface de jonction triangulaire pour laisser subsister une jonction 59''' en onglet sensiblement à angle droit par rapport à la bissectrice des coins de la partie centrale. On réalise ainsi, de manière techniquement plus simple, le même effet que celui décrit en relation avec le mode de réalisation illustré par les figures 5a à 5f.

On remarquera que dans le mode opératoire qui vient d'être décrit en relation avec ces figures 5a, 5b, 5c, 5g et 5h, les diverses opérations (solidarisation des élastiques, formation des découpes, pliage, collage) s'effectuent suivant le sens longitudinal de la bande. La conception de ce produit est donc bien adaptée au procédé de fabrication en continu de culottes à partir d'une bande de matériau souple.

Une variante du procédé de réalisation d'une couche-culotte présentant le même aspect que la culotte illustrée à la figure 5e, à la différence de la présence d'un coussin absorbant, est schématiquement représenté sur la figure 7.

Suivant ce mode de réalisation, une bande 73 d'un matériau perméable à l'humidité, par exemple une bande de non-tissé, destinée à constituer la face interne de la couche-culotte, est alimentée en continu à partir d'une bobine 74, et une bande 70 de matériau souple imperméable à l'humidité, par exemple de matière plastique, destiné à constituer la face externe de la couche-culotte, est alimentée en continu à partir d'une bobine 71, en même temps que des coussins absorbants 75 sont amenés de manière discontinue entre les deux bandes. Des élastiques 79, 79' déroulés en continu à l'état tendu de bobines 78, 78' sont périodiquement fixés à l'état tendu à la bande 70 en des emplacements situés latéralement de part et d'autre des emplacements destinés à recevoir les coussins 75. L'ensemble est alors solidarisé sous forme d'une bande de matériau composite 76 maintenue à l'état tendu. Pour la fixation des élastiques 78, 78', de même que pour la cadence d'introduction des coussins 75 et pour l'ensemble des opérations ultérieures, on détermine une longueur élémentaire de bande 75' correspondant à la longueur d'une ébauche nécessaire à la réalisation d'une couche-culotte, la bande étant alors considérée comme une succession d'ébauches, la limite entre les ébauches successives étant matérialisée en traits mixtes sur le dessin.

Dans la bande 76, on réalise ensuite, à hauteur de la partie centrale de chaque ébauche, deux découpes 77, 77' en forme d'échancrure en arc de cercle, généralement symétrique par rapport à l'axe longitudinal de la bande, pour donner à l'ébauche une forme générale en sablier. Ensuite, on déroule en continu de deux bobines 72, 72' des éléments élastiques 80, 80' à l'état tendu. Ces éléments élastiques sont constitués chacun d'une bande de matériau souple à laquelle sont fixés des élastiques à l'état tendu. Les éléments élastiques 80, 80' à l'état tendu sont superposés aux bords longitudinaux de la bande 76, et fixés aux ébauches que constitue cette bande, à chaque coin desdites ébauches. Comme dans le mode de réalisation des figures 5g et 5h, la fixation des éléments élastiques 80, 80' s'effectue par soudage en onglet par rapport aux coins des ébauches, c'est-à-dire sensiblement à angle droit par rapport à la bissectrice de chaque coin. Au cours de cette opération de fixation, l'excédent de matière soudée ou collée constituant les coins de l'ébauche est découpé et l'article terminé 81 est séparé de l'extrémité de la bande.

Il y a lieu de noter que les découpes 77, 77' en forme d'échancrures en arc de cercle peuvent être également omises, afin d'obtenir un article de conception plus simple, de forme rectangulaire. Un tel article est illustré sur la figure 10. Cet article se compose de deux éléments élastiques de ceinture 100, 100' fixés à une pièce plane 102 de forme rectangulaire, composée d'une feuille imperméable à l'humidité, d'un coussin absorbant et d'une feuille perméable à l'humidité. Les élastiques 103 des éléments de ceinture élastiques 100, 100' ainsi que des élastiques 104 fixés sur la pièce 102 pour assurer un serrage élastique du passage des jambes sont représentés en pointillés. Les zones triangulaires de fixation des éléments élastiques 100, 100' aux coins de la pièce 102 sont indiquées hachurées. Les passages pour les jambes sont formés entre les parties intermédiaires libres des éléments élastiques 100, 100' et la pièce 102.

Les figures 8a et 8b illustrent un mode de pliage de la couche-culotte terminée 81 de la figure 7, permettant, d'une part, un emballage individuel aisé et favorisant, d'autre part, un retournement de la couche-culotte pour l'amener dans l'état illustré à la figure 8c, c'est-à-dire dans un état où le matériau souple imperméable 70 constitue l'extérieur de la couche-culotte. En effet, à la suite du procédé illustré à la figure 7, si on relâche le produit terminé 81, la tension des éléments élastiques de ceinture 80, 80' qui se trouvent sur le dessus de la bande de matériau souple 70 a tendance à incurver le produit fini de telle sorte que la bande 70 constitue la face interne du produit. Pour éviter cet effet, on plie longitudinalement l'article terminé 81 pour former deux rabats 82, 82' latéraux que l'on amène suivant le sens des flèches 83, 83' sous la face constituée par la feuille perméable 73. Le relâchement des élastiques provoquera alors la courbure de l'article 81 de telle manière que la feuille perméable 73 constitue la face interne de l'article. Cette tendance sera encore favorisée, et l'emballage facilité, si l'article subit en outre un pliage transversal pour former deux rabats complémentaires 85, 85' (figure 8b).

Enfin, la figure 9 illustre schématiquement les étapes d'un procédé permettant l'obtention d'un autre type de culottes.

Suivant ce procédé, on forme dans la bande 90, dans les zones d'extrémités avant et arrière de chaque ébauche, des découpes en coin 91 pour constituer des pointes 92 aux emplacements des coins de pièce plane constituant la culotte. On rabat ensuite les coins 92 sur la bande 90, de sorte que les parties des coins de la pièce plane disposées normalement dans le sens transversal se retrouvent, suite au rabattement, orientées dans le sens longitudinal de la bande 90. On fixe ensuite, dans le sens longitudinal de la bande, des éléments élastiques 93, 93' à l'état tendu aux pointes rabattues 92. Enfin, on découpe dans la bande 90 la forme définitive de la pièce plane constituant la culotte, suivant le contour 94, et par le fait même on découpe l'excédent des éléments élastiques au-delà des pointes repliées, et on libère l'article obtenu. Conformément au procédé de l'invention, la bande 90 à laquelle sont fixés les éléments élastiques est maintenue à l'état tendu jusqu'à la réalisation complète de l'article terminé.

Il va de soi que l'on peut utiliser pour la feuille souple dans laquelle la culotte est réalisée tout matériau convenable, par exemple un matériau textile, tissé ou non tissé. Cependant, l'invention se prête tout particulièrement à la réalisation de culottes à jeter, en non-tissé. De même, vu sa simplicité de mise en oeuvre du procédé à l'aide de matériaux composites, et son prix de revient résultant de la cadence de production élevée qu'elle autorise, l'invention s'applique parfaitement à la réalisation d'articles d'hygiène tels des couches-culottes, des culottes d'incontinence pour adultes, et d'autres articles d'hygiène analogues. Lors de la réalisation de couches-culottes, qui présentent ici sous la forme de slips, il est avantageux de prévoir, dans la ceinture, des zones d'affaiblissement permettant la déchirure de la ceinture, afin de permettre une ouverture et un enlèvement conventionnels de la couche-culotte lorsque cette dernière est souillée.

## Revendications

1. Culotte en forme de slip à ceinture élastique, réalisée à partir d'une pièce plane de matériau souple, de forme générale rectangulaire et d'éléments élastiques fixés à cette pièce, caractérisée par le fait qu'elle comprend deux éléments élastiques (5, 5'; 35, 35'; 45, 45'; 55, 55'; 103) s'étendant, lorsque ladite pièce (1, 30, 40, 50, 102) de matériau souple est disposée à plat, à l'état tendu le long des deux bords longitudinaux opposés (4, 4'; 34, 34'; 44, 44'; 54, 54') de ladite pièce, les deux extrémités de chacun desdits éléments élastiques état solidaires de ladite pièce uniquement en des endroits situés au voisinage des deux extrémités transversales (4'', 4'''; 34'', 34'''; 54'', 54''') de cette dernière.

2. Culotte suivant la revendication 1, caractérisée par le fait que les deux éléments élastiques sont constitués par deux bandes (100, 100') rapportées sur la pièce de matériau souple, uniquement leurs deux extrémités étant fixées sur ladite pièce.

3. Culotte suivant la revendication 1, caractérisée par le fait que les deux éléments élastiques sont constitués par deux bandes longitudinales délimitées dans le matériau de ladite pièce par deux découpes longitudinales partielles (3, 3'; 33, 33'; 43, 43'; 53, 53') ménagées dans ladite pièce, entre les deux extrémités transversales de cette dernière, en retrait vers l'intérieur par rapport aux bords longitudinaux opposés de ladite pièce.

4. Culotte suivant l'une quelconque ces revendications précédentes, caractérisée par le fait que les éléments élastiques sont fixés à la pièce de matériau souple en onglet (59', 59''') par rapport aux coins de cette pièce.

5. Procédé de fabrication en continu de culottes en forme de slip à ceinture élastique à partir d'un matériau souple en bande, caractérisé par le fait qu'il consiste à:

alimenter en continu, à l'état tendu, au moins une bande (70, 73) d'un matériau souple,

alimenter en continu parallèlement au sens de défilement de la bande, de part et d'autre de l'axe médian longitudinal de cette dernière, des éléments élastiques (80, 80') à l'état tendu,

déterminer une longueur élémentaire de bande (75') correspondant à la longueur nécessaire à la confection d'une culotte, la bande étant alors considérée comme formant une succession d'ébauches,

solidariser de la bande à l'état tendu, les éléments élastiques à l'état tendu dans le sens de défilement de la bande, cette solidarisation s'effectuant uniquement en des endroits proches de l'extrémité arrière et de l'extrémité avant de chaque ébauche,

découper la bande et les éléments élastiques dans le sens transversal, dans la zone de séparation des ébauches successives.

6. Procédé de fabrication en continu de culottes en forme de slip à ceinture élastique, à partir d'un matériau souple en bande, caractérisé par le fait qu'il consiste à:

alimenter en continu, à l'état tendu, au moins une bande de matériau souple,

alimenter en continu parallèlement au sens de défilement de la bande, de part et d'autre de l'axe médian longitudinal de cette dernière, légèrement en retrait par rapport aux bords longitudinaux de la bande, des éléments élastiques à l'état tendu,

déterminer une longueur élémentaire de bande correspondant à la longueur nécessaire à la confection d'une culotte, la bande étant alors considérée comme formant une succession d'ébauches,

solidariser en continu de la bande à l'état tendu, les éléments élastiques à l'état tendu dans le sens de défilement de la bande,

former dans la bande, à hauteur de chaque ébauche, entre les deux extrémités de cette dernière, en retrait vers l'intérieur par rapport auxdits élastiques, deux découpes partielles symétriques orientées généralement suivant la direction longitudinale de la bande et ayant une longueur légèrement inférieure à la longueur de l'ébauche, et

découper la bande et les élastique dans le sens transversal, dans la zone de séparation des ébauches successives.

7. Procédé suivant la revendication 5 ou 6, caractérisé par le fait que les éléments élastiques sont solidarisés avec la bande, à chaque coin d'une ébauche, sensiblement en onglet par rapport aux coins.

8. Procédé suivant la revendication 5 ou 6, caractérisé par le fait qu'on forme dans la bande (90), dans les zones d'extrémité avant et arrière de chaque ébauche, de part et d'autre de l'axe médian longitudinal de la bande, des découpes en coin (91) pour former des pointes (92) aux emplacements des coins de la partie d'ébauche destinée à constituer la culotte, on replie sur la face supérieure de l'ébauche les pointes (92) ainsi formées, on solidarise les éléments élastiques (93, 93') à l'état tendu de la face apparente des pointes repliées, et on découpe dans la bande, dans chaque ébauche, la forme définitive (94) de la partie de matériau souple constituant la culotte.

9. Procédé suivant l'une quelconque des revendications 5 à 7, caractérisé par le fait qu'on solidarise de la bande (70), sur une partie de la longueur de chaque ébauche, sensiblement à mi-distance entre les extrémités de chaque ébauche, des longueurs limitées d'élastiques (79, 79') à l'état tendu, disposés généralement suivant la direction longitudinale de la bande, au voisinage des bords longitudinaux de la partie centrale de matériau souple constituant la culotte.

10. Procédé suivant la revendication 6, caractérisé par le fait qu'on rabat (57, 57') les bords longitudinaux de la bande, pourvus d'élastiques (55, 55') et situés à l'extérieur des découpes (53, 53'), sur la partie centrale de l'ébauche, et on solidarise en onglet (59'), de la partie centrale de l'ébauche, les parties des rabats situées entre les extrémités des découpes et les bords transversaux correspondants de l'ébauche.

**Claims**

1. Pants in the form of briefs with an elastic belt, made from a planar piece of flexible material of generally rectangular shape and of elastic members fastened to this piece, characterized in that it comprises two elastic members (5, 5'; 35, 35'; 45, 45'; 55, 55'; 103) extending, when the said piece (1, 30, 40, 50, 102) of flexible material is arranged flat, in the stretched state along the two opposite lengthwise edges (4, 4'; 34, 34'; 44, 44'; 54, 54') of the said piece, the two ends of each of the said elastic members being firmly fixed to the said piece solely in places situated in the vicinity of the two transverse ends (4'', 4'''; 34'', 34'''; 54'', 54''') of the latter.

2. Pants according to claim 1, characterized in that the two elastic members consists of two strips (100, 100') added onto the piece of flexible material, only the two ends being fastened to the said piece.

3. Pants according to claim 1, characterized in that the two elastic members consists of two lengthwise strips bounded in the material of the said piece by two partial lengthwise cuts (3, 3'; 33, 33'; 43, 43', 53, 53') arranged in the said piece between the two transverse ends of the latter, set back inwards relative to the opposite lengthwise edges of the said piece.

4. Pants according to any one of the preceding claims, characterized in that the elastic members are fastened to the piece of flexible material bias (59', 59''') relative to the corners of this piece.

5. Process for the continuous manufacture of pants in the form of briefs with an elastic belt, from a piece of flexible material in strip form, characterized in that it consists in:

continuously feeding, in the stretched state, at least one strip (70, 73) of a flexible material,

continuously feeding elastic members (80, 80') in the stretched state, in the direction of movement of the strip, on both sides of the lengthwise median axis of the latter,

determining an elementary strip length (75') corresponding to the length needed to manufacture a pair of pants, the strip being then considered as forming a sequence of blanks,

firmly fixing the elastic members in the stretched state in the direction of movement of the strip to the strip in the stretched state, this firm fixing being performed solely in places close to the rear end and front end of each blank,

sectioning the strip and the elastic members in the transverse direction in the region separating the successive blanks.

6. Process for the continuous manufacture of pants in the form of briefs with an elastic belt, from a flexible material in strip form, characterized in that it consists in:

continuously feeding, in the stretched state, at least one strip of flexible material,

continuously feeding elastic members in the stretched state, the direction of movement of the strip, on both sides of the lengthwise median axis of the latter, slightly set back relative to the lengthwise edges of the strip,

determining a unit strip length corresponding to the length needed for the manufacture of a pair of pants, the strip being then considered as forming a sequence of blanks,

continuously firmly fixing the elastic members in the stretched state in the direction of movement of the strip to the strip in the stretched state,

forming in the strip, at the level of each blank, between the two ends of the latter, set back inwards relative to the said elastics, two symmetrical partial cuts oriented generally in the lenghtwise direction of the strip and having a length which is slightly shorter than the length of the blank, and

sectioning the strip and the elastics in the transverse direction in the region separating the successive blanks.

7. Process according to claim 5 or 6, characterized in that the elastic members are firmly fixed to the strip, at each corner of a blank, substantially bias relative to the corners.

8. Process according to claim 5 or 6, characterized in that angular cuts (91) are made in the strip (90) in the front and rear regions of each blank, on both sides of the lengthwise median axis of the strip to form tongues (92) at the locations of the corners of the part of a blank intended to form the pants, the tongues (92) thus formed are folded back onto the upper face of the blank, the elastic members (93, 93') are firmly fixed in the stretched state to the apparent face of the folded tongues, and the definitive shape (94) of the part of flexible material forming the pants is cut from the strip, in each blank.

9. Process according to any one of claims 5 to 7, characterized in that limited lengths of elastics (79, 79') in the stretched state, arranged generally in the lengthwise direction of the strip, in the vicinity of the lengthwise edges of the central part of flexible material forming the pants, are firmly fixed to the strip (70) over a part of the length of each blank, substantially midway between the ends of each blank.

10. Process according to claim 6, characterized in that the lengthwise edges of the strip, which are provided with elastics (55, 55') and situated outside the cuts (53, 53') are folded back (57, 57') onto the central part of the blank and the parts of the fold-back flaps situated between the ends of the cuts and the corresponding transverse edges of the blank are firmly fixed bias (59') to the central part of the blank.

**Patentansprüche**

1. Hose in Slipform mit elastischem Bund, hergestellt aus einem ebenen Stück aus flexiblem Werkstoff von im allgemeinen rechteckiger Form und aus an diesem Stück befestigten, elastischen Elementen, dadurch gekennzeichnet, daß sie zwei elastische Elemente (5, 5'; 35, 35'; 45, 45'; 55, 55'; 103) besitzt, die sich, wenn das Stück (1, 30, 40, 50, 102) aus weichem Werkstoff flach aufgelegt ist, in gespanntem Zustand entlang der beiden gegenüberliegender Längsränder (4, 4', 34, 34'; 44, 44'; 54, 54') des Stückes erstrecken, wobei die beiden Enden jedes elastischen Elementes mit dem Stück nur an Stellen verbunden ist, die in der Nähe der beiden Querenden (4", 4'''; 34", 34'''; 54", 54''') des letzteren angeordnet sind.

2. Hose nach Anspruch 1, dadurch gekennzeichnet, daß die beiden elastischen Elemente von zwei Bändern (100, 100') gebildet werden, die am Stück aus weichem Werkstoff angesetzt sind, wobei auf dem genannten Stück nur ihre beiden Enden befestigt sind.

3. Hose nach Anspruch 1, dadurch gekennzeichnet, daß die beiden elastischen Elemente von zwei länglichen Bändern gebildet werden, die im Werkstoff des genannten Stückes durch zwei im Stück zwischen den beiden Querenden des letzteren vorgesehene, längslaufende Teilschnitte (3, 3'; 33, 33'; 43, 43'; 53, 53') begrenzt werden, die gegenüber den einander gegenüberliegenden Längsrändern des Stückes nach innen versetzt sind.

4. Hose nach einem der vorhergehenden Ansprüche, dadurch gekennzeichnet, daß die elastischen Elemente am Stück aus flexiblem Werkstoff schräg (59', 59''') zu den Ecken dieses Stückes befestigt sind.

5. Verfahren zur kontinuierlichen Herstellung von Hosen in Slipform mit elastischem Bund, ausgehend von einem bahnförmigen, flexiblem Werkstoff, dadurch gekennzeichnet, daß es besteht aus:

kontinuierlichem Zuführen wenigstens einer Bahn (70, 73) aus flexiblem Werkstoff in gespanntem Zustand,

kontinuierlichem Zuführen von elastischen Elementen (80, 80') in gespanntem Zustand parallel zur Bewegungsrichtung der Bahn und beiderseits der Längsmittelachse der letzteren,

Bestimmen einer Elementlänge der Bahn (75') entsprechend der Länge, die für die Herstellung einer Unterhose notwendig ist, wobei die Bahn dann als eine Folge von Zuschnitten bildend betrachtet wird,

Verbinden von in Richtung der Bewegung der Bahn gespannten, elastischen Elementen mit der gespannten Bahn, wobei diese Verbindung lediglich in Stellen ausgeführt wird, die nahe dem vorderen und hinteren Ende jedes Zuschnittes sind, und

Abschneiden der Bahn und der elastischen Elemente in Querrichtung im Bereich der Teilung zwischen aufeinanderfolgenden Zuschnitten.

6. Verfahren zur kontinuierlichen Herstellung von Hosen in Slipform mit elastischem Bund, ausgehend von einem bahnförmigen, flexiblem Werkstoff, dadurch gekennzeichnet, daß es besteht aus:

kontinuierlichem Zuführen wenigstens einer Bahn aus flexiblem Werkstoff in gespanntem Zustand,

kontinuierlichem Zuführen von elastischen Elementen in gespanntem Zustand parallel zur Bewegungsrichtung der Bahn und, gegenüber den Längsrändern der Bahn etwas zurückversetzt beiderseits der Längsmittelachse der letzteren,

Bestimmen einer Elementlänge der Bahn entsprechend der Länge, die für die Herstellung einer Hose notwendig ist, wobei die Bahn dann als eine Folge von Zuschnitten betrachtet wird,

kontinuierlichem Verbinden der Bahn in gespanntem Zustand mit in Richtung der Bewegung der Bahn gespannten, elastischen Elementen,

Ausführen von zwei gegenüber den elastischen Elementen nach innen versetzten Teilschnitten im Bereich jedes Zuschnittes zwischen den beiden Enden des letzteren, die symmetrisch im allgemeinen in der Längsrichtung der Bahn ausgerichtet sind und die eine Länge haben, die etwas kleiner ist als die Länge eines Zuschnittes, und

Abschneiden der Bahn und der elastischen Elemente in Querrichtung im Bereich der Teilung aufeinanderfolgender Zuschnitte.

7. Verfahren nach Anspruch 5 oder 6, dadurch gekennzeichnet, daß die elastischen Elemente mit der Bahn in jeder Ecke eines Zuschnittes im wesentlichen schräg zu den Ecken verbunden werden.

8. Verfahren nach Anspruch 5 oder 6, dadurch gekennzeichnet, daß man in der Bahn (90) im Bereich des vorderen und des hinteren Endes jedes Zuschnittes beiderseits der Längsmittelachse der Bahn im Bereich der Ecken des Teils des Zuschnittes, der dazu bestimmt ist, die Unterhose zu bilden, eckige Schnitte (91) ausführt, um Spitzen (92) zu bilden, daß man die so gebildeten Spitzen (92) auf die Oberseite des Zuschnittes umschlägt, daß man die elastischen Elemente (93, 93') in gespanntem Zustand mit der sichtbaren Seite der umgefalteten Spitzen verbindet und daß man in der Bahn in jedem Zuschnitt die endgültige Form (94) des die Hose bildenden Teiles des flexiblen Werkstoffes zuschneidet.

9. Verfahren nach einem der Ansprüche 5 bis 7, dadurch gekennzeichnet, daß man mit der Bahn (70) auf einem Teil der Länge jedes Zuschnittes im wesentlichen in der Mitte zwischen den Enden jedes Zuschnittes begrenzte Längen elastischer Elemente (79, 79') im gespannten Zustand befestigt, die im allgemeinen in der Längsrichtung der Bahn, in der Nähe der Längsränder des Mittelteils des weichen Werkstoffes, der die Unterhose bildet, angeordnet sind.

10. Verfahren nach Anspruch 6, dadurch gekennzeichnet, daß man die Längsränder (57, 57') der Bahn, die mit elastischen Elementen (55, 55') versehen und außerhalb der Schnitte (53, 53') angeordnet sind, auf den mittleren Teil des Zuschnittes umschlägt und daß man die Teile der Umschläge, die zwischen den Enden der Schnitte und den entsprechenden Querrändern des Zuschnittes angeordnet sind, schräg (59) mit dem mittleren Teil des Zuschnittes verbindet.

FIG. 1

FIG. 2

FIG. 3

FIG. 4

FIG. 6

FIG. 5g

FIG. 5h

FIG. 5a

FIG. 5b

FIG. 5c

FIG. 5d

FIG. 5e

FIG. 5f

FIG.7

FIG.8a

FIG.8b

FIG.8c

FIG.9

FIG.10